Europäisches Patentamt

European Patent Office

Office européen des brevets

(11) Publication number: **0 315 715 B1**

## EUROPEAN PATENT SPECIFICATION

(45) Date of publication of patent specification: **03.06.92**  (51) Int. Cl.⁵: **B01J 29/28**, C07C 1/20, C07C 15/00

(21) Application number: **87309901.4**

(22) Date of filing: **09.11.87**

(54) **The use of dehydroxylated zeolites in the catalytic.**

(43) Date of publication of application:
**17.05.89 Bulletin 89/20**

(45) Publication of the grant of the patent:
**03.06.92 Bulletin 92/23**

(84) Designated Contracting States:
**BE DE FR GB IT NL**

(56) References cited:
**EP-A- 0 123 449**
**EP-A- 0 152 233**
**FR-A- 2 312 478**
**US-A- 3 929 668**

(73) Proprietor: **MOBIL OIL CORPORATION**
**150 East 42nd Street**
**New York New York 10017(US)**

(72) Inventor: **Chang, Clarence Dayton**
**11 Murray Place**
**Princeton New Jersey 08540(US)**
Inventor: **Hellring, Stuart Damon**
**29 Carlton Avenue**
**Trenton New Jersey 08618(US)**
Inventor: **Socha, Richard Francis**
**32 Colleen Circle**
**Trenton New Jersey 08638(US)**

(74) Representative: **Colmer, Stephen Gary**
**Patent Department c/o Mobil Services Company Limited Mobil Court 3 Clements Inn**
**London WC2A 2EB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to the use of low acidity zeolites in xylene isomerization.

Zeolitic materials, both natural and synthetic, are known to have the capability for catalysing various types of hydrocarbon conversion reactions which take place in the presence of catalytic sites with acidic functionality. These zeolite materials generally have ordered, porous crystalline structures within which there are a number of small cavities that are interconnected by a number of still smaller channels. These cavities and channels are precisely uniform in size within a specific zeolitic material. Since the dimension of these pores are such as to accept for adsorption purposes molecules of certain dimensions, while rejecting those of larger dimension, these materials have commonly been known to be "molecular sieves" and are utilized in a variety of ways to take advantage of the adsorptive propertis of these compositions. The structures may be determined by X-ray diffraction techniques.

These molecular sieves include a wide variety of positive ion-containing crystalline aluminosilicates and other siliceous materials such as borosilicates, ferrosilicates and gallosilicates in which the presence of the trivalent metal at sites within the silicate structure provides the desired acidic functionality in an environment which permits access to the site only by molecules of appropriate size so that the acid catalyzed reactions are carried out in a "shape selective" manner. Metal cations such as sodium which are usually present in these materials when they are synthesized may be converted to the hydrogen form by exchange with ammonium ions followed by heating to drive off ammonia or by direct exchange with an acid such as hydrochloric acid, if the zeolite is not degraded by the acid. Useful catalysts are also produced by a combination of ion-exchange treatments in which the crystalline silicate may be converted to the acid form and then may be ion exchanged with a solution of various metal salts to produce the metal exchanged zeolite.

The acid activity of aluminosilicate zeolites may be so high that conventional hydrocarbon conversion processes and apparatus cannot take full advantage of this high activity. For example, in catalytic cracking, high activity may yield excessive coke formation and the production of large anions of light gases. The acid activity of zeolite catalysts may, however, be lowered to a level at which the use of such catalysts in catalytic conversions is satisfactory and, in fact, results in a considerable increase in the efficiency of such processes. Reactions which have been performed successfully over low acidity zeolites include the conversion of oxygenates such as methanol and dimethyl ether to olefins and other hydrocarbon products, xylene isomerization, aromatic alkylation, and olefin oligomerization, as well as catalytic dewaxing and hydrodewaxing and hydroisomerization.

One method of reducing the activity of aluminosilicate zeolite catalyst is by compositing the zeolite with a matrix material which is relatively inactive. Suitable matrix materials include inorganic oxides, such as those of silica, zirconia, alumina, magnesia and combinations of such materials with one another, as well as clays and other refractory materials.

Other methods to reduce the activity of acid zeolites include cation exchange with sodium or other alkali metal cations or by forming the zeolites with high silica:alumina mole ratios in the structure or framework. An important method in reducing the activity of zeolite catalysts is by a process of steam treating. By controlled steaming, it is possible to produce zeolite catalysts having any desired degree of activity. The degree of steaming of a specified catalyst to achieve a desired activity level is largely dependent upon the nature of the zeolite. Steam treatment, however, often requires long periods of time to treat the catalyst effectively for activity reduction.

U.S. Patent No. 3,939,058 discloses methods of modifying the catalytic properties of zeolites. One such method is calcination which is defined as heating at high temperatures but below the sintering temperature of the zeolite for varying periods of time. Other methods are also disclosed, including compositing the zeolite in a matrix and steam treatment. The patent further discloses that the crystallinity retention of catalysts may be improved by precalcination of the crystalline aluminosilicate. For example, the patent states that it has been found possible to preserve the crystallinity of aluminosilicates such as the rare earth exchanged synthetic faujasites, by calcining the zeolite to drive off water, thereby forming a more suitable structure and minimzing loss in crystallinity during subsequent rapid drying, as in spray drying, wet processing, steaming and aging. The calcining may be accomplished by heating the crystalline aluminosilicate sieve after ion exchange to a temperature below the sintering temperature of the sieve and generally in the range of from 500° to 1600° F (about 260° to 870° C).

Similarly, U.S. Patent No. 4,141,859 discloses a method of controlling the relative acid activity of zeolite catalysts, by treating the zeolitic component with air or steam at elevated temperatures, e.g., up to 1700° F (about 925° C) in air or at temperatures from about 800° F to about 1700° F (about 425° C to 925° C).

Calcination of freshly synthesized zeolites to remove adsorbed water and organic materials that have been used to form the zeolite crystals is necessary to activate the zeolite and accordingly has generally been employed. Also, as stated above, precalcination of the zeolite has been found to stabilize the crystallinity of the zeolite. However, heat treatment may remove hydroxyl groups from the framework of the zeolite. Thus, dehydroxylation of a decationized Y zeolite is discussed in Zeolite Chemistry and Catalysts, ACS Monograph 171, pages 142 and 143, in which dehydroxylation of Y zeolite is stated to result from prolonged calcination at relatively high temperatures, resulting finally in the structural collapse of the zeolite and the formation of an amorphous silica or silica-alumina structure. For these reasons, the use of high temperatures has generally been avoided in zeolite synthesis. When organic materials are to be removed from the freshly synthesized zeolite, temperatures of about 1000°F (about 540°C) are typical and generally not exceeded in order to avoid damage to the crystal structure.

U.S. Patent No. 4,016,218 discloses a process for reducing the acitivy of a zeolite, such as ZSM-5, to an alpha value less than 250, preferably less than 200 but greater than 10, by thermal treatment in an inert atmosphere at 650-980°C (1200-1800°F) or in steam at 260-980°C (500-1800°F). The resultant, thermally treated zeolite is found to be useful for alkylating aromatics with olefins.

According to the present invention, it is now been found that zeolites having a silica/alumina molar ratio of at least 12 and a constraint index of 1 to 12 are particularly useful in xylene isomerization, in that they exhibit improved selectivity to the para-isomer, after having been thermally treated at a temperature of at least 725°C in a water-free atmosphere to reduce their alpha value to below 100.

The present invention, therefore, resides in a process for isomerising a xylene feed to increase the concentration of the para-isomer, comprising contacting the feed with a catalyst comprising a crystalline aluminosilicate zeolite which has a silica/alumina ratio of at least 12 and a constraint index of 1 to 12 and which has been thermally modified by heating the zeolite at a temperature of at least 725°C in an essentially water-free atmosphere to reduce its alpha value to below 100.

The zeolites employed in the xylene isomerization process of the invention have a silica/alumina molar ratio of at least 12 and a constraint index of 1 to 12. Non-limiting examples of such zeolites include ZSM-5, ZSM-11, ZSM-12, ZSM-23, ZSM-35, and ZSM-48.

The synthesis and characteristics of zeolite ZSM-5 are described in U.S. Patent No. 3,702,886; of zeolite ZSM-11 in U.S. Patent No. 3,709,979; of zeolite ZSM-12 in U.S. Patent No. 3,832,449; of zeolite ZSM-23 in U.S. Patent No. 4,076,842; of zeolite ZSM-35 in U.S. Patent No. 4,016,245 and of zeolite ZSM-48 in U.S. Patent No. 4,397,827.

The above-defined zeolites can function as catalysts even when modified to have low alpha values, typically less than 10, and even at alpha values substantially lower than 1. As noted above, low acid activity has previously been achieved by using zeolites of very high silica/alumina ratio, extensive ion exchange of the zeolite with sodium or other alkali metal cations, or by severe temperature steaming of zeolites.

Alpha activity is an approximate indication of the catalytic cracking activity of the catalyst compared to a standard catalyst, a highly active silica-alumina cracking catalyst taken as alpha = 1. Alpha activity is further defined in U.S. Patent No. 3,354,078 and the Journal of Catalysis, Vol. 4, pages 522-529, August, 1965. The Constraint Index of a zeolite may be determined by the method described in U.S. Patent No. 4,016,218.

Generally, ZSM-5 and other intermediate pore size zeolites having a Constraint Index of 1-12 are activated by calcining the zeolite at temperatures of about 550°C to remove water and organic directing agents which are typically included in the synthesis mixture from which the zeolite is made. In accordance with present invention, however, the zeolites are treated at or above 725°C and preferably at or above 800°C (but less than the sintering temperature of the zeolite) to reduce the acid activity of the zeolite.. Although the temperature required for reducing the alpha activity may vary between the individual zeolites the treatment temperature should generally not exceed 1100°C, above which temperature the structural framework begins to collapse. Preferably, the zeolite retains at least 55% of its crystallinity after the heat treatment.

Calcination is achieved by heating the zeolite at the desired elevated temperature, in dry air, hydrogen or an inert gas such as nitrogen. Typically, the heat treatment proceeds for at least 1 hour, although heating may last between 1-24 hours. The heat treatment is dry (no water), although up to 2% by weight steam may be included in the calcining atmosphere.

It is theorized that at the high temperature used for achieving reduced alpha activity, removal of framework hydroxyl groups occurs (i.e. dehydroxylation). The heat treatment should not continue beyond the point at which crystallinity of the zeolite is lost.

The initial alpha value of the zeolite used in the present process is preferably 200 or more, whereas the final alpha value of the zeolite is below 100 and in many cases below 50. Alpha values even lower than this may nonetheless be possessed by catalytically useful materials in reactions requiring only a limited degree

EP 0 315 715 B1

of acidity.

The following Examples illustrate the invention. All samples of the thermally deactivated ZSM-5 produced in Examples 1-4 originated from the $NH_4$ form ZSM-5 ($SiO_2/Al_2O_3$ = 70), which had been converted into the hydrogen form by calcination at 1°C/min. to 538°C, then held for 10 hr. to produce a zeolite having an alpha of 214.

Example 1

An HZSM-5 sample was calcined at 800°C for 1 hour, then at 1000°C for 1 hour (alpha = 0.4).

Example 2

An HZSM-5 sample was calcined at 1000°C for 1 hour, (alpha = 0.9).

Example 3

An HZSM-5 sample was calcined at 800°C for 1 hour, (alpha = 12). A mixture of this zeolite (65%) in $Al_2O_3$ was used.

Example 4

An HZSM-5 sample, as a mixture (65%) in $Al_2O_3$, was calcined at 1038°C for 16 hours (alpha = 0.1).

Example 5

The conversion of m-xylene over the thermally treated ZSM-5 of Example 2 (alpha = 0.9) is shown in Table 1.

4

TABLE 1

| | Feed | | |
|---|---|---|---|
| LHSV | | 6.8 | 1 |
| Temperature, °F (°C) | | 427 (220) | 427 (220) |
| Pressure, psig (kPa) | | 150 (1135) | 150 (1135) |
| Time on Stream (Hours) | | 0.5 | 4.5 |
| **% Products** | | | |
| Toluene | -- | 0.08 | 0.95 |
| P | 0.14 | 8.49 | 23.35 |
| m　　Xylene | 99.86 | 87.59 | 58.81 |
| o | -- | 3.51 | 15.46 |
| $C_9$+ Aromatics | -- | 0.25 | 1.429 |
| Normalized | -- | | |
| **Xylenes** | | | |
| P | -- | 8.52 | 23.92 |
| m　　Xylene | -- | 87.95 | 60.24 |
| o | -- | 3.51 | 15.84 |
| **p-Xylene** | | | |
| % Equilibrium* | -- | 36.3 | 101.8 |
| % m-Xylene Converted | -- | 12.27 | 41.05 |

* Xylenes equilibrium at 427°C: 23.5 para, 52.1 meta, 24.4 ortho.

Example 6

Table 2 illustrates xylene isomerization over thermally deactivated ZSM-5.

### TABLE 2

| Catalyst (α) | | | 0.9 | 0.2 |
|---|---|---|---|---|
| Pressure, psig (kPa) | | | 150 (556) | 150 (556) |
| Temperature, °F (°C) | | | 800 (427) | 800 (427) |
| LHSV | | | 6.8 | 1 |
| Time on Stream (Hours) | | | 3 | 2 |
| **Products wt.%** | **Feed** | | | |
| Benzene | -- | | 0.15 | 1.56 |
| Toluene | -- | | 0.06 | 1.03 |
| Ethylbenzene | 14.79 | | 14.02 | 11.38 |
| para | 0.24 | | 7.25 | 19.51 |
| meta    Xylene | 84.41 | | 75.38 | 54.89 |
| ortho | 0.56 | | 2.29 | 9.23 |
| $C_9$+ Aromatics | -- | | 0.85 | 2.40 |
| **Normalized Xylenes** | | | | |
| para | | | 8.54 | 23.33 |
| meta    Xylene | | | 88.77 | 65.63 |
| ortho | | | 2.70 | 11.04 |
| **para-Xylene** | | | | |
| % Equilibrium* | | | 36.3 | 99.3 |
| % m-Xylene Conv. | | | 10.7 | 35.0 |
| % Ethylbenzene Conv. | | | 5.3 | 23.1 |

* Xylenes equilibrium at 427°C, p-xylene 23.5, m-xylene 52.1, o-xylene 24.4

Example 7

Two ZSM-5 samples (5g each, silica/alumina = 72, alpha = 179), identified herein as (a) and (b), were subjected respectively to high temperature calcination and steaming as follows:

(a) One sample was calcined in a box furnace under flowing nitrogen by heating at 21°C/min to 1000°C, then holding 1 hr at 1000°C. The sample was removed from the furnace and rapidly cooled under a blanket of flowing argon. The sample had an alpha of 6.8. A small portion (1g) of the sample was then exchanged with aqueous ammonium hydroxide (1M) overnight at room temperature after being treated with ammonia gas. Temperature programmed ammonia desorption (TPAD) indicated that the sample had a silica/alumina ratio of about 290.

(b) The other sample was treated with flowing steam (water partial pressure = 1 atm, 101 kPa) at 600°C for 13.5 hr. The resulting sample had an alpha value of 6.1 and a silica/alumina ratio by TPAD of 250.

Each of the treated ZSM-5 samples was then pelleted, meshed (14/30) and loaded into a stainless steel reactor and heated under flowing nitrogen at a temperature of 426°C at a pressure of 150 psig (1135 kPa), whereupon meta-xylene was pumped as a neat liquid at varying flow rates into the reactor. In the case of the calcined sample (a), the selectivity of the conversion from meta- to paraxylene rose from a value of about 48% at a meta-xylene conversion level of about 48% to a value of about 80% (99.9% xylenes in product) at a conversion level of 20%. In the case of the steamed sample (b) the para-selectivity also increased as the conversion level was decreasd from 48% but now the para-selectivity was only 65% at 20% conversion.

**Claims**

1. A process for isomerising a xylene feed to increase the concentration of the para-isomer, comprising contacting the feed with a catalyst comprising a crystalline aluminosilicate zeolite which has a silica/alumina ratio of at least 12 and a constraint index of 1 to 12 and which has been thermally modified by heating the zeolite at a temperature of at least 725°C in an essentially water-free atmosphere to reduce its alpha value to below 100.

2. A process according to claim 1 in which the zeolite has been heated to a temperature of at least 800°C.

3. A process according to claim 1 or claim 2 in which the alpha value of the zeolite is reduced to less than 50.

4. A process according to any preceding claim in which the initial alpha value of the zeolite is at least 150.

5. A process according to any preceding claim in which the zeolite is ZSM-5.

**Revendications**

1. Un procédé pour isomériser une charge de xylènes de façon à augmenter la concentration de l'isomère para, comprenant la mise en contact de la charge avec un catalyseur comprenant une zéolite de type aluminosilicate cristalline qui a un rapport molaire silice/alumine d'au moins 12 et un indice de contrainte de 1 à 12 et qui a été thermiquement modifié par chauffage de la zéolite à une température d'au moins 725°C dans une atmosphère essentiellement exempte d'eau pour réduire sa valeur alpha à moins de 100.

2. Un procédé suivant la revendication 1, dans lequel la zéolite a été chauffée à une température d'au moins 800°C.

3. Un procédé suivant la revendication 1 ou la revendication 2, dans lequel la valeur alpha de la zéolite est réduite à moins de 50.

4. Un procédé suivant l'une quelconque des revendications précédentes, dans lequel la valeur alpha initiale de la zéolite est d'au moins 150.

5. Un procédé suivant l'une quelconque des revendications précédentes, dans lequel la zéolite est une ZSM-5.

**Patentansprüche**

1. Verfahren zur Isomerisierung einer Xylol-Zufuhr, um die Konzentration des p-Isomers zu erhöhen, das den Kontakt der Zufuhr mit einem Katalysator umfaßt, der einen kristallinen Aluminosilikat-Zeolith umfaßt, der ein Siliciumdioxid/Aluminiumoxid-Verhältnis von mindestens 12 und einen Zwangsindex von 1-12 aufweist und der thermisch modifiziert wurde, indem der Zeolith bei einer Temperatur von mindestens 725°C in einer im wesentlichen wasserfreien Atmosphäre erwärmt wurde, um dessen $\alpha$-Wert auf unter 100 zu reduzieren.

2. Verfahren nach Anspruch 1, worin der Zeolith auf eine Teperatur von mindestens 800°C erwärmt

7

wurde.

3. Verfahren nach Anspruch 1 oder 2, worin der $\alpha$-Wert des Zeoliths auf weniger als 50 reduziert wurde.

4. Verfahren nach einem der vorstehenden Ansprüche, worin der ursprüngliche $\alpha$-Wert des Zeoliths mindestens 150 beträgt.

5. Verfahren nach einem der vorstehenden Ansprüche, worin der Zeolith ZSM-5 ist.